# EUROPÄISCHE PATENTSCHRIFT

(11) **EP 2 919 671 B1**
(45) Veröffentlichungstag und Bekanntmachung des Hinweises auf die Patenterteilung: **27.06.2018**
(21) Anmeldenummer: 13799492.7
(22) Anmeldetag: 07.11.2013
(51) Int. Cl.: A61B 17/16

(54) **MARKRAUMBOHRER**
MEDULLARY CAVITY DRILL
ALÉSOIR MÉDULLAIRE

(30) Priorität: 13.11.2012 DE 102012110888
(43) Veröffentlichungstag der Anmeldung: 23.09.2015
(73) Patentinhaber: Trokamed GmbH, 78187 Geisingen (DE)
(72) Erfinder: TRÖNDLE, Karlheinz, 78187 Geisingen (DE)
(74) Vertreter: Patentanwälte und Rechtsanwalt Weiß, Arat & Partner mbB
(86) Internationale Anmeldenummer: PCT/EP2013/073312
(87) Internationale Veröffentlichungsnummer: WO 2014/075990

(56) Entgegenhaltungen:
- WO-A1-98/49948
- WO-A1-2005/094693
- US-A- 4 473 070
- US-A1- 2006 015 110
- US-A1- 2011 144 649

## Beschreibung

Die Erfindung betrifft einen Markraumbohrer nach dem Oberbegriff des Anspruchs 1.

### STAND DER TECHNIK

Aus dem Stand der Technik sind verschiedene Arten von Markraumbohrern bekannt. Hierbei ist nachteilig, dass der Bohrkopf nur sehr umständlich ausgetauscht werden kann. Ausserdem ist nachteilig, dass ein fehlerfreier Austausch des Bohrkopf nur besonders geschultem Personal möglich ist.

In diesem Zusammenhang wir auf die WO 2005/094693 A1 hingewiesen, welche einen Markraumbohrer offenbart, welcher einen austauschbaren Bohrerkopf aufzeigt. Daneben wird auf die US 2011/0144649 A1 und die US 2006/0015110 A1 hingewiesen, welche ebenfalls Markraumbohrer mit austauschbaren Bohrerköpfen offenbart. Lediglich der Vollständigkeit halber wird auf die US 4,473,070 A und die WO 98/49948 A1 hingewiesen, welche ebenfalls Markraumbohrer nach dem Stand der Technik offenbaren.

### AUFGABE

Aufgabe der Erfindung ist es die Nachteile des Standes der Technik abzustellen und ausserdem einen Markraumbohrer mit einem wechselbaren Bohrkopf zur Verfügung zu stellen, der eine einfache und schnelle Verbindung zulässt und hierbei Fehler in der Handhabung möglichst ausschliesst.

### LÖSUNG DER AUFGABE

Zur Lösung der Aufgabe führen die Merkmale des Anspruchs 1.

Ein erfindungsgemässer Markraumbohrer besteht aus einem Bohrkopf und einer Welle, wobei der Bohrkopf in die Welle ein- und/oder ausschiebbar ist. Dies bedeutet im Einzelnen, dass der Bohrkopf teilweise in die Welle eingreifend eingeschoben werden kann. Es kann aber auch bedeuten, dass die Welle in den Bohrkopf eingeschoben werden kann. Hierzu kann der Bohrkopf ein männliches Bestandteil oder ein weibliches Bestandteil umfassen. In gleicher Weise kann die Welle ein weibliches Bestandteil oder ein männliches Bestandteil umfassen. Die Auswahl, ob der Bohrkopf oder die Welle ein männliches bzw. ein weibliches Bestandteil umfasst, ist daran auszurichten, dass die Welle und der Bohrkopf miteinander verbunden werden können. Die Verbindung zwischen dem Bohrkopf und der Welle wird im Folgenden auch als Konnektierung bezeichnet. Vorteilhaft an dieser Ausführung ist der Umstand, dass schnell und einfach eine Konnektierung zwischen dem Bohrkopf und der Welle hergestellt werden kann. Das Zusammenspiel von männlichen und weiblichem Bestandteil erlaub ein einfaches Ineinanderschieben des Bohrkopf und der Welle.

Beim erfindungsgemässen Markraumbohrer weist die Welle proximal eine rinnenförmige Ausnehmung mit einem Bodenbereich auf. Dieser Bodenbereich ist bevorzugt eben ausgebildet. Von diesem Bodenbereich gehen die Seitenwände in proximaler Richtung verjüngend hinzu Abschlusskanten der rinnenförmigen Ausnehmung, die proximalseitig wiederum konisch zueinander verlaufend ausgebildet sind. Durch diese vom Bodenbereich proximal zusammenlaufenden Seitenwände der rinnenförmigen Ausnehmung sowie die Abschlusskanten der rinnenförmigen Ausnehmung, die wiederum konisch zueinander verlaufend ausgebildet sind, wird ein besonderer Raum geschaffen, in den ein männlicher Bestandteil des Bohrkopfs oder je nach Ausführung der Welle nur auf eine Art und Weise richtig eingeschoben werden kann. Dies führt vorteilhaft dazu, dass beim Aufschieben des Bohrkopfes auf die Welle bzw. des Einschiebens des Bohrkopfs in die Welle weniger Fehler passieren können, da es lediglich eine einzige mögliche Form der Konnektierung gibt. Erfindungsgemäß weist der Markraumbohrer an dem Bohrkopf distal ein Schienenelement mit einem Oberflächenbereich auf. Dieser Oberflächenbereich ist derart ausgebildet, dass er mit dem Bodenbereich zusammenwirken kann. Dies bedeutet im Einzelnen, dass der Bodenbereich und der Oberflächenbereich ohne grössere Reibung aufeinander gleiten können. Der Oberflächenbereich weist eine Senkung auf, welche mit einer Kugelarretierung des Bodenbereichs zusammenwirkt. Dies wiederum bedeutet, dass die Kugelarretierung bei einem Erreichen eines definierten Punktes die zunächst niedergedrückte Kugelarretierung freigibt, so dass die Kugelarretierung in die Senkung hineingedrückt wird. Die Kugelarretierung ist hierbei bevorzugt in der Art ausgebildet, dass eine federgelagerte Kugel in den Bodenbereich eingelassen ist. Wenn dann das Schienenelement in die rinnenförmige Ausnehmung geschoben wird, wird zunächst die Kugel in den Bodenbereich hineingedrückt, bis die Senkung über der Kugelarretierung zum Stehen kommt. Danach wird die Kugelarretierung wieder aus dem Bodenbereich heraus in die Senkung hineingedrückt, so dass der Bohrkopf mit der Welle gesichert verbunden ist. Dies wird als Wirkverbindung zwischen der Senkung und der Kugelarretierung bezeichnet. Hierdurch wird vorteilhaft erreicht, dass eine zusätzliche Sicherung der Verbindung zwischen dem Bohrkopf und der Welle vorhanden ist. Ein versehentliches Herausgleiten des Bohrkopfs aus der Welle ist nicht möglich.

Ein erfindungsgemässer Markraumbohrer ist ausserdem derart ausgebildet, dass das Schienenelement distalseitig konisch zueinander verlaufende Abschlusskanten aufweist. Zwischen diesen Abschlusskanten ist der Oberflächenbereich ausgeformt.

Weiterhin können der Bohrkopf mit einer Teilfingermulde und die Welle mit einer weiteren Teilfingermulde versehen sein. Beim ordnungsgemässen Aufbringen des Bohrkopfs auf die Welle bzw. des Einschiebens des Bohrkopfes in die Welle entsteht eine Fingermulde. Hierbei ist vorteilhaft, dass durch die Fingermulde auf einfache Art und Weise festgestellt werden kann, ob der Bohrkopf richtig auf die Welle aufgebracht wurde.

### FIGURENBESCHREIBUNG

Weitere Vorteile, Merkmale und Einzelheiten ergeben sich aus der nachfolgenden Beschreibung eines Ausführungsbeispiels. Das Ausführungsbeispiel ist anhand von Figuren beschrieben. Die Figuren zeigen im Einzelnen in
Figur 1 eine Seitenansicht eines erfindungsgemässen Markraumbohrers in entkoppelter Stellung;
Figur 2 eine Seitenansicht eines Markraumbohrers in gekoppelter Stellung;
Figur 3 eine vergrösserte Seitenansicht einer erfindungsgemässen Welle;
Figur 4 eine vergrösserte Ansicht eines Bohrkopfs.

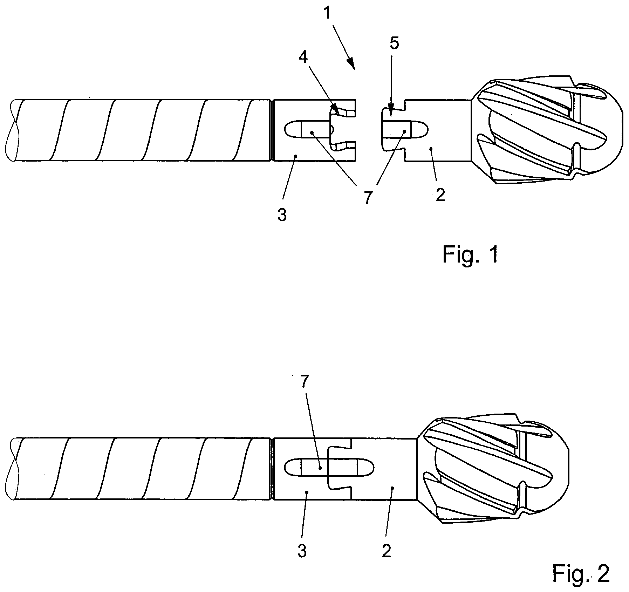

In Figur 1 ist ein Wechselsystem 1 gezeigt. Dieses Wechselsystem 1 besteht in erster Linie aus dem Bohrkopf 2 und der Welle 3, die in diesem Ausführungsbeispiel als flexible Welle ausgeführt ist. Ausserdem ist in Figur 1 gezeigt, wie die Welle 3 einen weiblichen Bestandteil 4 aufweist und der Bohrkopf 2 einen männlichen Bestandteil 5 aufweist. Ausserdem ist eine Fingermulde 7 gezeigt, die sich als Teilfingermulde auf der Welle 3 und dem Bohrkopf 2 befindet.

In Figur 2 ist gezeigt, wie der Bohrkopf 2 mit der Welle 3 gekoppelt ist. Bei dieser Ansicht fällt auf, dass die Fingermulde 7 nun komplettiert ist.

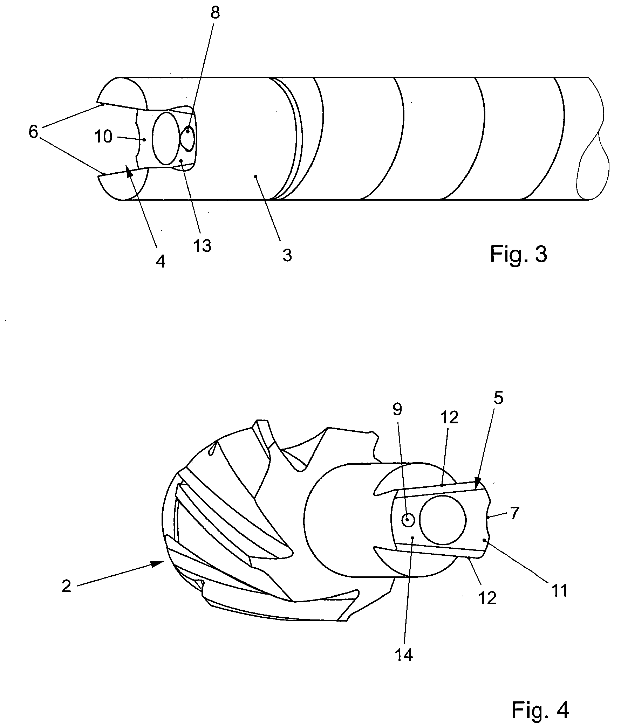

In Figur 3 ist die Welle 3 nochmals gezeigt. Dort ist zu erkennen, wie der weibliche Bestandteil 4 aus einer rinnenförmigen Ausnehmung 10 und zwei Abschlusskanten 6 besteht. Ausserdem ist gezeigt, wie in einem Bodenbereich 13 eine Kugelarretierung 8 vorhanden ist. Die Abschlusskanten 6 verlaufen konisch zueinander. In gleicher Weise ist auch der Bodenbereich 13 konisch geformt. Allerdings weist der Bodenbereich 13 eine grössere Fläche auf als der zwischen den beiden Abschlusskanten 6 vorhandene Freiraum. Dies erklärt sich dadurch, dass von dem Bodenbereich 13 hin zu den Abschlusskanten 6 eine Verjüngung stattfindet.

In Figur 4 ist der Bohrkopf 2 in vergrösserter Ansicht von schräg hinten gezeigt. Dort ist zu erkennen, wie der männliche Bestandteil 5 aus einem Schienenelement 11 besteht. Das Schienenelement 11 weist in diesem Ausführungsbeispiel distalseitig konisch zueinander verlaufende Abschlusskanten 12. Ausserdem weist das Schienenelement 11 auf seinem distalseitigen Oberflächenbereich 14 eine Senkung 9 auf. Ausserdem ist eine Teilfingermulde 7 in der Figur 4 teilweise zu erkennen.

### Positionszahlenliste

- 1.: Wechselsystem
- 2.: Bohrkopf
- 3.: Welle
- 4.: Weiblicher Bestandteil
- 5.: Männlicher Bestandteil
- 6.: Abschlusskante
- 7.: Fingermulde
- 8.: Kugelarretierung
- 9.: Senkung
- 10.: rinnenförmige Ausnehmung
- 11.: Schienenelement
- 12.: Abschlusskante
- 13.: Bodenbereich
- 14.: Oberflächenbereich

## Patentansprüche

1. Markraumbohrer mit einem Bohrkopf (2) und einer Welle (3), wobei der Bohrkopf (2) in die Welle (3) ein- und/oder ausschiebbar ist, wobei der Bohrkopf (2) einen männlichen Bestandteil (5) und die Welle (3) einen weiblichen Bestandteil (4) umfasst oder der Bohrkopf (2) einen weiblichen Bestandteil und die Welle (3) einen männlichen Bestandteil umfasst, wobei der weibliche Bestandteil aus einer rinnenförmigen Ausnehmung (10) mit einem Bodenbereich (13) besteht und der männliche Bestandteil distal aus einem Schienenelement (11) mit einem Oberflächenbereich (14) besteht, wobei das Schienenelement (11) in die rinnenförmige Ausnehmung (10) ein- und/oder ausschiebbar ist,
**dadurch gekennzeichnet, dass** Abschlusskanten (6) der rinnenförmigen Ausnehmung (10); proximalseitig konisch zueinander verlaufend ausgebildet sind und Abschlusskanten (12) des Schienenelements (11) distalseitig konisch zueinander verlaufen, wobei der Oberflächenbereich (14) eine Senkung (9) aufweist und der Bodenbereich (13) eine Kugelarretierung (8) umfasst, wobei die Senkung (9) mit der Kugelarretierung (8) in Wirkverbindung steht.

## Claims

1. A medullary cavity drill having a drill head (2) and a shaft (3), wherein the drill head (2) can be slid into and/or out of the shaft (3), wherein the drill head (2) comprises a male component (5) and the shaft (3) comprises a female component (4) or the drill head (2) comprises a female component and the shaft (3) comprises a male component, wherein the female component consists of a groove-shaped recess (10) having a base area (13) and the male component distally consists of a rail element (11) having a surface area (14), wherein the rail element (11) can be slid into and/or out of the groove-shaped recess (10),
**characterised in that**
terminating edges (6) of the groove-shaped recess (10) are designed to extend conically to one another on the proximal side and terminating edges (12) of the rail element (11) extend conically to one another on the distal side, wherein the surface area (14) has a depression (9) and the base area (13) comprises a ball detent (8), wherein the depression (9) is operatively connected to the ball detent (8).

## Revendications

1. Alésoir médullaire avec une tête de forage (2) et un arbre (3), dans lequel la tête de forage (2) peut être introduite dans et/ou sortie de l'arbre (3), dans lequel la tête de forage (2) comporte un composant mâle (5) et le arbre (3) comporte un composant femelle (4) ou la tête de forage (2) comporte un composant femelle et l'arbre (3) comporte un composant mâle, dans lequel le composant femelle consiste en un évidement en forme de rainure (10) avec une zone de fond (13) et le composant mâle consiste distalement en un élément de rail (11) avec une zone de surface (14), dans lequel l'élément de rail (11) peut être introduit dans et/ou sorti de l'évidement en forme de rainure (10),
**caractérisé par le fait que** les bords terminaux (6) de l'évidement en forme de rainure (10) sont réalisés du côté proximal de manière à s'étendre coniquement l'un vers l'autre et que les bords terminaux (12) de l'élément de rail (11) s'étendent du côté distal coniquement l'un vers l'autre, la zone de surface (14) présentant un creux (9) et la zone de fond (13) comportant un verrouillage à billes (8), le creux (9) étant en liaison active avec le verrouillage à billes (8).
